(19) Europäisches Patentamt
European Patent Office
Office européen des brevets

(11) Publication number: **0 202 441**
**B1**

(12) # EUROPEAN PATENT SPECIFICATION

(45) Date of publication of patent specification: **17.11.88**

(21) Application number: **86104605.0**

(22) Date of filing: **04.04.86**

(51) Int. Cl.⁴: **C 07 D 487/04,** A 61 K 31/55 // (C07D487/04, 243:00, 235:00)

(54) **Oxadiazolylimidazobenzodiazepines, process for their preparation and pharmaceutical compositions.**

(30) Priority: **17.05.85 DK 2204/85**
**12.08.85 DK 3659/85**
**17.10.85 DK 4769/85**
**20.12.85 DK 5994/85**
**06.01.86 US 816732**

(43) Date of publication of application:
**26.11.86 Bulletin 86/48**

(45) Publication of the grant of the patent:
**17.11.88 Bulletin 88/46**

(84) Designated Contracting States:
**AT BE CH DE FR GB IT LI LU NL SE**

(56) References cited:
**EP-A-0 109 921**
**EP-A-0 150 040**

(73) Proprietor: **A/S FERROSAN**
**Sydmarken 5**
**DK-2860 Soeborg (DK)**

(72) Inventor: **Wätjen, Frank**
**Josteinsvej 27**
**DK-2880 Bagsvaerd (DK)**
Inventor: **Engelstoft, Mogens**
**Mosegaard Park 121**
**DK-3500 Vaerloese (DK)**
Inventor: **Hansen, John Bondo**
**Havretoften 10**
**DK-2800 Lyngby (DK)**
Inventor: **Jensen, Leif Helth**
**Boegehoej 37**
**DK-2900 Hellerup (DK)**

(74) Representative: **Patentanwälte Grünecker, Kinkeldey, Stockmair & Partner**
**Maximilianstrasse 58**
**D-8000 München 22 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 202 441 B1

## Description

BACKGROUND OF INVENTION

2Field of Invention

This invention relates to novel oxadiazolyl imidazobenzodiazepine compounds, pharmaceutical compositions thereof, and to methods of preparing such compounds. The novel compounds are useful in psychopharmaceutical applications, e.g. in the treatment of central nervous system ailments, for example, as an anticonvulsant or an anxiolytic.

*Prior Art*

It is well known (Squires, R. F. and Braestrup, C., Nature (London) *266,* (1977) 732) that specific sites in the central nervous systems of vertebrates exhibit a high specific affinity for binding 1,4- and 1,5-benzodiazepines. These sites are called benzodiazepine receptors.

EP—A—109,921 (published May 30, 1984) and corresponding US—A—4,507,313 (filed November 15, 1983, issued March 26, 1985 and Danish patent application No. 5102/82 disclose compounds having the general formula I

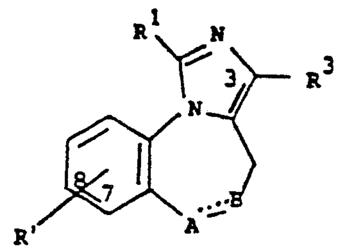

(I)

wherein R′ is hydrogen, chlorine, fluorine, or nitro in the 7- or 8-position,

$R^1$ is hydrogenj or lower-alkyl of up to 3 carbon atoms,

$R^3$ is an oxadiazolyl of the formula

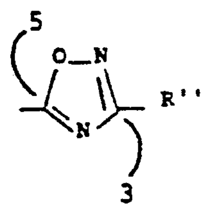

or

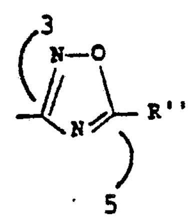

R″ is lower-alkyl of up to 3 carbon atoms,

A . . . . . B is a group of the formula

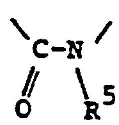

or

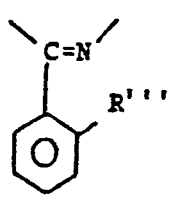

$R^5$ is hydrogen or methyl

R‴ is hydrogen or chlorine.

EP—A—109,921 further discloses (page 2, line 17 through page 3, line 3) that such oxadiazolyl benzodiazepines of EP—A—27,214 (US—A—4,316,839) page 7, line 4 and oxadiazolyl beta-carbolines, as disclosed in earlier EP—B—54,507 (USP 4,435,403), exhibit stronger binding affinity for the benzodiazepine receptors than the analogous substituted compounds which are alkyl esters (rather than such oxadiazolyl derivatives).

EP—A—150 040 (published July 31, 1985) and corresponding Danish patent application 245/85 (made available July 22, 1984) also disclose oxidiazol imidazobenzodiazepines.

The disclosure of EP—A—150040 is very broad. Its disclosure of 1,2,4-oxadiazolyl-benzodiazepine compounds can be illustrated by Formulas II and III.

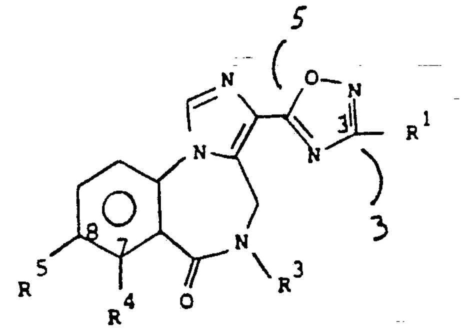

II

(4 Ring Structure)

wherein

$R^1$ = alkyl, cycloalkyl, methoxymethyl

$R^3$ = H, $CH_3$, and

$R^4$, $R^5$ = H, halogen

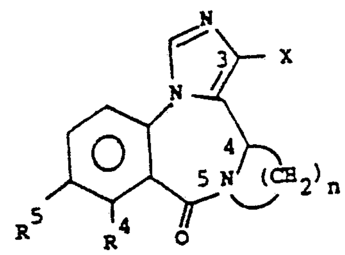

III

(5 Ring Structure)

wherein

X = 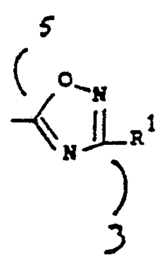 and 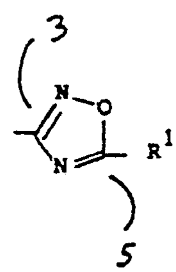

wherein

$R^1$ = alkyl, cycloalkyl, $CF_3$, or methoxymethyl

$R^4$, $R^5$ = H, halogen, $CF_3$, and

n = 2 or 3.

EP—A—150040 includes no examples of compounds having the formula IV.

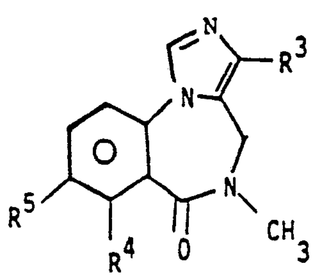

(IV)

wherein $R^3$ is

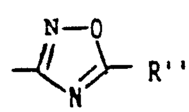 ,

and much less wherein $R^3$ is

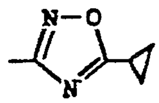

nor does this application disclose compounds of above formula IV wherein any of $R^4$ or $R^5$ is cyano, lower alkyl or trifluoromethyl and further no compounds wherein $R^4$ is F, cyano, lower alkyl or trifluoromethyl and of course much less compounds of formula IV wherein $R^3$ is

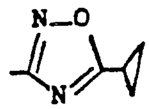

and $R^4$ is Cl, Br, F, $CF_3$, $CH_3$ or CN.

The compounds of EP—A—150040, examples 2, 3, 16, and 43 are old compounds EP—A—109921, page 3, lines 4—5, and examples 2 and 3 are preferred compounds of EP—A—150040, page 5, lines 34—37, and Danish patent application No. 245/85 page 5, lines 14—17.

The compounds of EP—A—150040, examples 2, 3, 16, 29, 32, 43, 44, 45, 49, 50, 51, 52, 53, and 56 are 5,6-dihydro-6-oxo-4H-imidazo(1,5-a)(1,4)benzodiazepine compounds. (Compounds of Formula II).

The compounds of EP—A—150040, examples 1, 8, 9, 17, 18, 23, 30 are 10, 11, 12, 12a-tetrahydro-9-oxo-9H-imidazo-(1,5-a)azeto(2,1-c)(1,4-benzodiazepine compounds. (Compounds of Formula III).

The compounds of EP—A—150040, examples 4, 5, 6, 7, 10, 11, 12, 13, 14, 15, 19, 20, 21, 22, 24, 25, 26, 27, 28, 31, 33, 34, 35, 36, 37, 38, 39, 40, 41, 42, 46, 48, 54, 55, 57, 58 and 59 are 11, 12, 13, 13a-tetrahydro-9-oxo-9H-imidazo-(1,5-a)pyrrolo(2,1-c)(1,4-benzodiazepine compounds. (Compounds of Formula III).

The compound of EP—A—150040, example 47 is an 11,13a-dihydro-9-oxo-9H-imidazo(1,5-a)pyrrolo(2,1-c)(1,4)benzodiazepine compound.

The compounds of EP—A—150040, examples 11, 15, 26, and 40 are 1,2,4-oxadiazol-3-yl compounds.

The compounds of EP—A—150040, examples 11, 15, 26, and 40 are 1,2,4-oxadiazol-3-yl compounds combined with an 11, 12, 13, 13a-tetrahydro-9-oxo-9H-imidazo(1,5-a)pyrrolo(2,1-c)(1,4)benzodiazepine skeleton (compounds of Formula III).

The compounds of EP—A—150040, example 40 is a 5-cyclopropyl-1,2,4-oxadiazol-3-yl compound with an 11, 12, 13, 13a-tetrahydro-9-oxo-9H-imidazo(1,5-a)pyrrolo(2,1-c)(1,4)benzodiazepine skeleton (compound of Formula III).

The compound of EP—A—150040, example 40 is a 5-cyclopropyl-1,2,4-oxidazol-3-yl compound which has been found grossly inferior in pharmacological and biochemical evaluations as further reported hereinafter.

The combined prior art of the 5,6-dihydro-6-oxo-4H-imidazo (1,5-a)(1,4)benzodiazepine compounds (compounds of formula II) is illustrated below.

The full EP-A-150040 disclosure of 1,2,4 oxadiazol-5-yl and 3-yl-5-methyl-imidazo (1,5-a) (1,4) benzodiazepine.

*

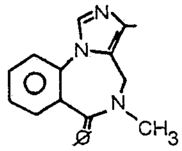
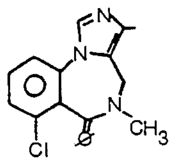
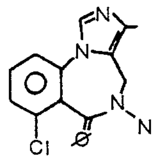
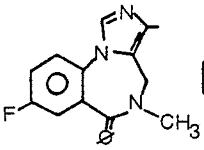
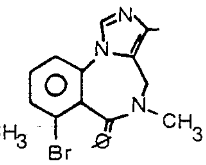

**

1,2,4-oxadiazol-5-yl

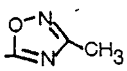

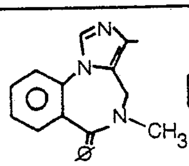
P3, *43*

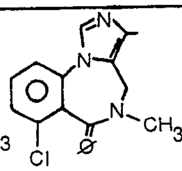
P1, *3*

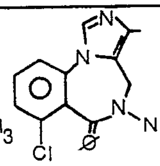
P1, *16*

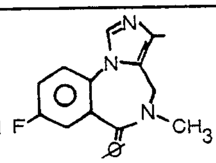
P1, *2*

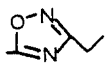

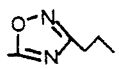

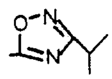

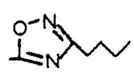

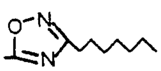

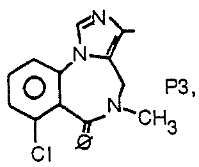
P3, *53*

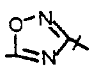

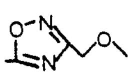

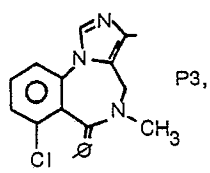
P3, *52*

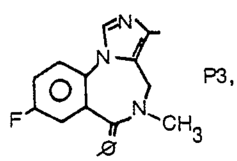
P3, *51*

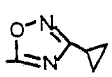

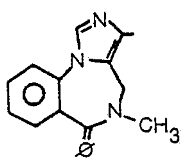
P3, *44*

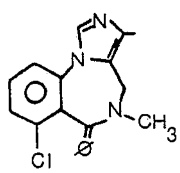
P2, *29*

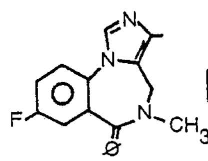
P2, *32*

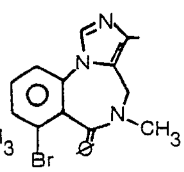
P3, *45*

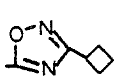

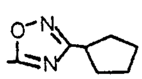

1,2,4-oxadiazol-3-yl

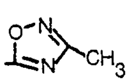

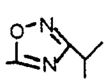

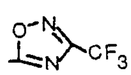

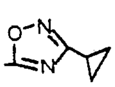

P1: examples 1–28 Priority Jan. 19, 1984 CH 225/'84
P2: " 29–41 Priority June 29, 1984 CH 3149/'84
P3: " 42–58 Priority Oct. 26, 1984 CH 5123/'84

*X* = example No. X
Framed areas: EP-A-109921
Blank areas: No disclosure in EP-A-150040

Of specific interest to the present invention EP—A—150040 discloses the following compounds

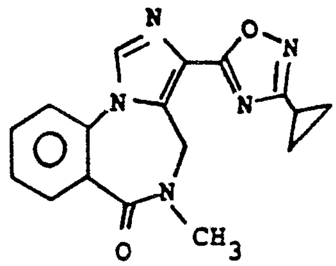

a 3-cyclopropyl-1,2,4-oxadiazol-5-yl compound

EXAMPLE 44

and

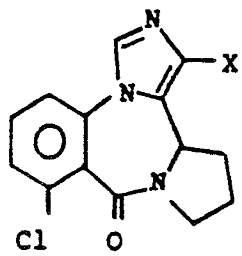

X = 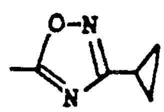

EXAMPLE 31

a 3-cyclopropyl-1,2,4-oxadiazol-5-yl compound

X = 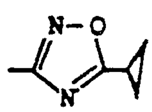

EXAMPLE 40

a 5-cyclopropyl-1,2,4-oxadiazol-3-yl compound

EP—A—150040 further claims a process for the preparation of such compounds (II and III) by reacting compounds of Formula V and VI

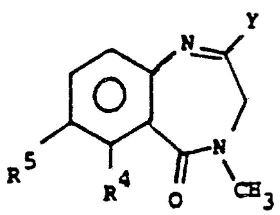

V

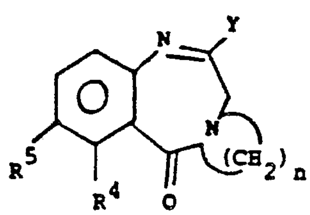

VI

wherein $R^4$, $R^5$ and n have the meanings set forth in the foregoing and Y is a leaving group, with a compound of the formula

$$CN—CH_2—x$$

wherein X has the meaning set forth in the foregoing for Formula III.

All oxadiazoles in EP—A—150040 are actually made by reacting intermediates V or VI with CN—$CH_2$—$CO_2R$ to form a compound of Formula II or III having the —$CO_2R$ substituent instead of an oxadiazol-ring, which compound is thereafter, in several steps, converted to an oxadiazole.

The new compounds provided by the present invention have the same type of structure and activity as disclosed in the prior art, but the particular and specific compound of the present invention, and the particular and specific 'subject matter as a whole', including not only its chemical structure but also its pharmacological properties, has been found to be both advantageous and unobvious from the standpoint of one skilled in the art.

OBJECTS

It is an object of the present invention to provide the novel compounds having the formula (VII)

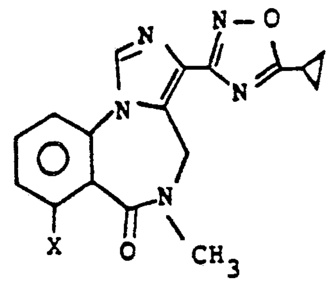

VII

wherein X is Cl, Br, F, $CF_3$, $CH_3$ or CN and pharmaceutically acceptable acid addition salts thereof, which are useful in the treatment of central nervous system disorders or ailments, especially as anticonvulsants and anxiolytics, a process for producing the same, and pharmaceutical compositions thereof.

SUMMARY OF THE INVENTION

The invention, in summary, comprises the following: A compound selected from the group consisting of 3-(5-cyclopropyl-1,2,4-oxadiazol-3-yl)-5,6-dihydro-5-methyl-6-oxo-7-X-4H-imidazo(1,5-a)(1,4)benzodiazepine and a pharmaceutically acceptable acid addition salt thereof; a pharmaceutical composition suitable for use in the treatment of central nervous system ailments, especially convulsions and anxiety states, comprising an effective amount of 3-(5-cyclopropyl-1,2,4-oxadiazol-3-yl)-5,6-dihydro-5-methyl-6-oxo-7-X-4H-imidazo(1,5-a)(1,4)benzodiazepine or a pharmaceutically acceptable acid addition salt thereof.

The invention also comprises a method of synthesizing the compound of the invention.

The free basic compounds of the present invention have the formula

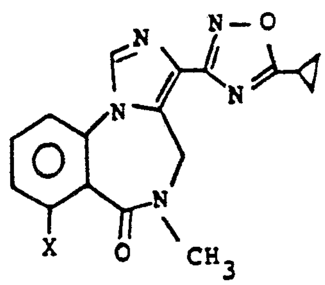

Mp.
X = Cl — 165,1—169.2°C
X = Br — 212—213°C
X = F — 188,2—189.0°C
X = $CF_3$ — 200,1°C
X = $CH_3$ — 175.0—175.5°C
X = CN — 237—9°C

These compounds can be prepared either by conventional methods analogous to the methods described in EP—A—109921 Example 3, EP—A—150040 Example 40, or EP—A—54507 Example 70 as illustrated below

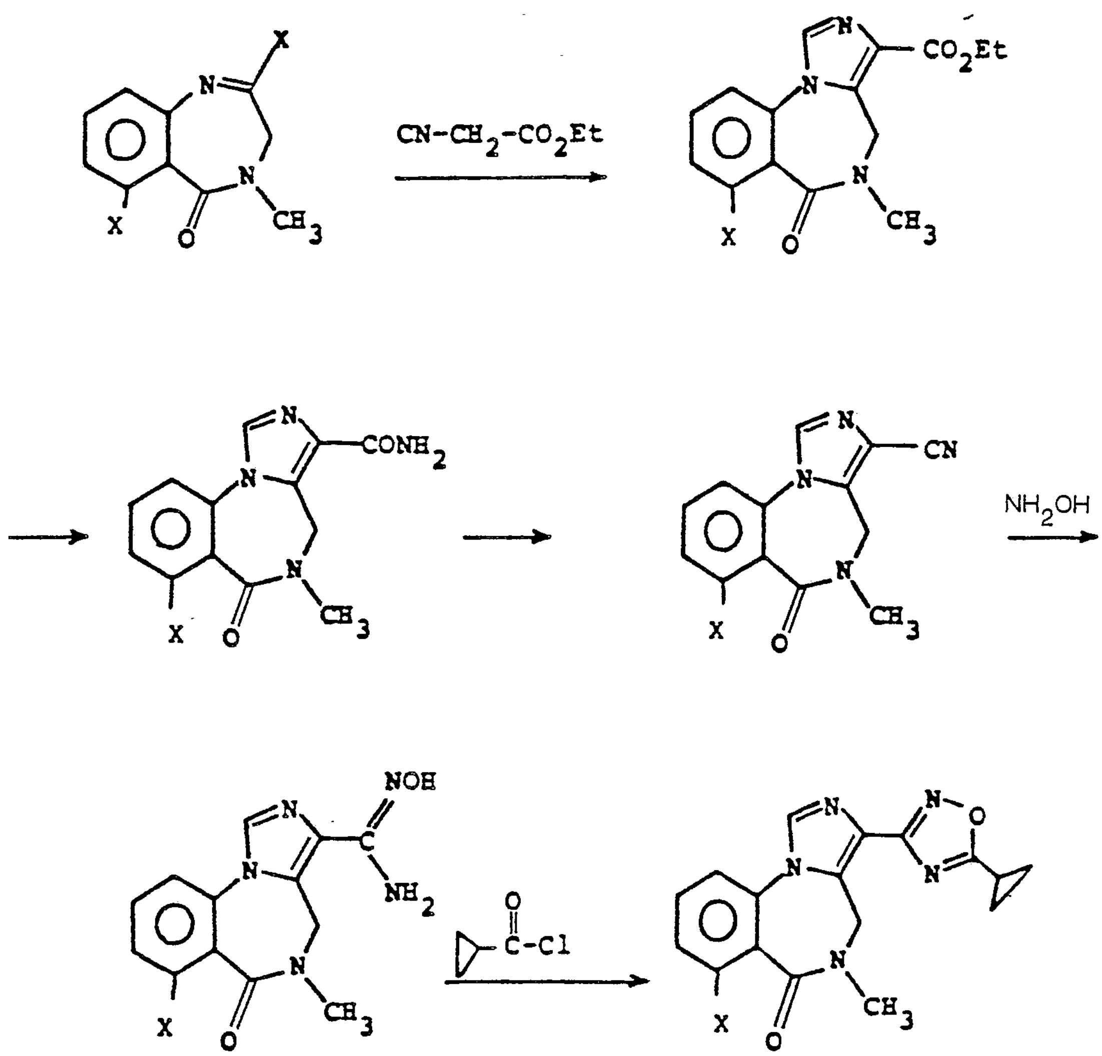


or by the new method provided by the present invention and as illustrated below

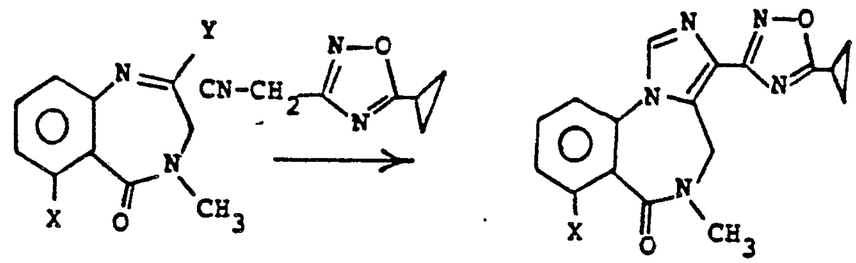

wherein

Y is a leaving group, such as the —OP(O)(O-ethyl)$_2$ group of Example 6 hereof. Alternatively, the leaving group may be any disclosed in US—A—4,031,079 or US—A—4,359,420, for example, halogen, alkylthio, e.g., methylthio, aralkylthio, N-nitrosoalkylamino, alkoxy, mercapto, —OP(O)(OR)$_2$ wherein R is lower-alkyl or —OP(O)(NR′R″) wherein R′ and R″ each represents lower-alkyl, or phenyl, or together with the nitrogen atom to which they are attached represent a heterocyclic radical such as morpholino, pyrrolidino, piperidino, or methylpiperazino. The reaction is preferably carried out under alkaline conditions, i.e., in the presence of a base, and among bases alkali metal, e.g., potassium or sodium,

alkoxides or hydrides are preferred. The reaction is preferably conducted in the presence of an organic solvent which is nonreactive with the reactants and products of reaction under the conditions of reaction, especially an anhydrous solvent and preferably an anhydrous aprotic solvent such as dimethylformamide (DMF) or the like. The temperature range employed may be any range suitable for the reaction to proceed at a reasonable rate and without undue delay or decomposition and a range from a −40°C to about room temperature is accordingly usually particularly suitable.

PHARMACEUTICAL COMPOSITIONS

The compound of the invention, together with a conventional adjuvant, carrier, or diluent, and if desired in the form of a pharmaceutically acceptable acid addition salt thereof, may be placed into the form of pharmaceutical compositions and unit dosages thereof, and in such form may be employed as solids, such as tablets, of filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective central nervous system ailment alleviating amount of the active ingredient commensurate with the intended daily dosage range to be employed. Tablets containing 10 mg of active ingredient or, more broadly, 10 to 30 mg, per tablet, are accordingly suitable representative unit dosage forms.

The compound of this invention can thus be used for the formulation of pharmaceutical preparations, e.g., for oral and parenteral administration to mammals including humans, in accordance with conventional methods of galenic pharmacy.

Conventional excipients are such pharmaceutically acceptable organic or inorganic carrier substances suitable for parenteral or oral application which do not deleteriously react with the active compound.

Examples of such carriers are water, salt solutions, alcohols, polyethylene glycols, polyhydroxy-ethoxylated castor oil, gelatin, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, such as luricants, preservatives, stabilizers, wetting agents, emulsifiers, salt for influencing osmotic pressure, buffers and/or coloring substances and the like, which do not deleteriously react with the active compound.

For parenteral application, particularly suitable are injectable solutions or suspensions, preferably aqueous solutions with the active compound dissolved in polyhydroxylated castor oil.

Ampoules are conveniently unit dosages.

For oral application, particularly suitable are tablets, dragees, or capsules having talc and/or a carbohydrate carrier or binder or the like, the carrier preferably being lactose and/or corn starch and/or potato starch. A syrup, elixir or the like can be used when a sweetened vehicle can be employed. Generally, as to broader ranges, the compound of the invention is dispensed in unit dosage form comprising 0,05—100 mg in a pharmaceutically acceptable carrier per unit dosage.

METHOD OF TREATING

Due to its high degree of affinity for the benzodiazepine receptors, the compounds of the invention are extremely useful in the treatment of central nervous system ailments or disorders, when administered in an amount effective for the alleviation, amelioration, or elimination thereof. The important CNS activity of the compounds of the invention includes both anticonvulsant and anxiolytic activities along with a low toxicity, together presenting a most favourable therapeutic index. The compounds of the invention may accordingly be administered to a subject, e.g., a living animal body, including a human, in need of the same for the treatment, alleviation, amelioration, or elimination of an indication, associated with the central nervous system and the so-called benzodiazepine receptors, which requires such psychopharmaceutical treatment, e.g. especially convulsion and/or anxiety states, if desired in the form of a pharmaceutically-acceptable acid addition salt thereof (such as the hydrobromide, hydrochloride or sulfate, in any event prepared in the usual or conventional manner, e.g., evaporation to dryness of the free base in solution together with the acid), ordinarily concurrently, simultaneously, or together with a pharmaceutically acceptable carrier or diluent, especially and preferably in the form of a pharmaceutical composition thereof, whether by oral, rectal, or parenteral (including subcutaneous) route, in an effective psychopharmaceutical central nervous system ailment alleviating amount, e.g., an anticonvulsant and/or anxiolytic amount, and in any event an amount which is effective for the alleviation of such a central nervous system ailment. Suitable dosage ranges are 1—200 milligrams daily, preferably 10—100 milligrams daily, and especially 30—70 milligrams daily, depending as usual upon the exact mode of administration, form in which administered, the indication toward which the administration is directed, the subject involved and the body weight of the subject involved, and the preference and experience of the physician or veterinarian in charge. Broader ranges for dosages of the compound according to this invention are 0.1—300 mg/day, preferable 1—30 mg/day, when administered to patients, e.g., humans, as a drug.

DETAILED DESCRIPTION OF THE INVENTION

The following Examples are given by way of illustration only, but are not to be construed as limiting.

Example 1

5,6-dihydro-5-methyl-6-oxo-7-chloro-4H-imidazo (1,5-a)(1,4)benzo-diazepine-3-carboxamide oxime

A mixture of 1.2 g 3-cyano-5,6-dihydro-5-methyl-6-oxo-7-chloro-4H-imidazo (1,5-a(1,4)benzodiazepine (prepared as in U.S.P. 4,316,839), 0.45 g of hydroxylamine hydrochloride, 20 ml of 99% ethanol, 2 ml water, and 1.2 g potassium carbonate was refluxed for 1½ hours. The reaction mixture was filtered and the filtrate was concentrated. The residue was treated with 50 ml of water and the crystalline solid was filtered off and washed with water.

M.p. 227.6—228.4°C.

Example 2

3-(5-cyclopropyl-1,2,4-oxadiazol-3-yl)-5,6-dihydro-5-methyl-6-oxo-7-chloro 4H-imidazo (1,5-a) (1,4)benzodiazepine.

A mixture of 580 mg 5,6-dihydro-5-methyl-6-oxo-7-chloro-4H-imidazo(1,5-a)(1,4)benzodiazepine-3-carboxamide oxime as prepared in Example 1 and 0.3 ml of cyclopropyl carboxylic acid chloride was stirred in 15 ml THF for two hours at 20°C and evaporated. After evaporation 20 ml of acetic acid was added and the mixture was refluxed for 2-½ hours and then evaporated. The reaction mixture was allowed to stand overnight at room temperature, whereafter the mixture was cooled, filtered, and the filtrate evaporated to give a residue as oily crystals. The residue was treated with ether to give the title compound as pale crystals which were collected by filtration.

M.p. 165—169°C.

Example 3

Formylaminomethyl-carboxamideoxime

To 53.6 g/0.638 mol/N-formylamino-acetonitrile* was added 0.55 mol freshly liberated hydroxylamine dissolved in 370 ml methanol. An ice bath was used to keep the temperature below 20°C during the addition. The solution was allowed to stand at room temperature overnight, whereafter it was evaporated to give the title compound as pale crystals.

Decomp. 104—110°C.

Example 4

3-Formylaminomethyl-5-cyclopropyl-1,2,4-oxadiazole

A mixture of 35 ml ethyl cyclopropylcarboxylate, 30 g formylamino-methyl-carboxamideoxime, 1 g sodium and 30 g crushed mol sieves (4Å) was refluxed in 300 ml abs. EtOH for 8 hours during which period further 1 g sodium was added. The reaction mixture was filtered and the filtrate was evaporated. The dark oily residue was suspended in 300 ml $CHCl_3$, filtered and the filtrate was evaporated to give the title compound as an oil.

H—NMR (60 MHz, $CDCl_3$) δ (ppm): 1.2 (4 H, m), 2.8 (1 H, m), 4.5 (2 H, d, j=6 Hz), 7.8 (1 H, broad-NH), 8.2 (1 H, s).

Example 5

5-Cyclopropyl-3-isocyanomethyl-1,2,4-oxadiazole

A stirred solution of 5-cyclopropyl-3-formylamino-methyl-1,2,4-oxadiazole (60 mmol) and triethylamine (176 mmol) in $CH_2Cl_2$ (100 ml) was charged dropwise with $POCl_3$ (60 mmol) at 0°C, whereafter a solution of $Na_2CO_3$ (60 mmol) in $H_2O$ (50 ml) was added. The mixture was heated to room temperature, whereafter the organic phase was separated, dried and evaporated *in vacuo.* The residue was treated with ether, decanted and the solution was evaporated to give the title compound as an oil.

The oil was processed without any further purification.

IR: $cm^{-1}$: 2160.

Example 6

3,4-Dihydro-4-methyl-6-cyano-2H-1,4-benzodiazepine-2,5(1H)-dione

2.3 g of 3,4-dihydro-4-methyl-6-bromo-2H-1,4-benzodiazepine-2,5(1H)-dione and 1.26 g of cupro cyanide was dissolved in 5 ml dimethyl formamide. This mixture was heated to 110°C for 30 minutes. This mixture was then cooled to 50°C and 2.4 g sodium cyanide in 6 ml water was added. The resulting mixture was then stirred for 10 minutes, whereafter 30 ml water was added. This mixture was extracted two times with 25 ml ethyl acetate. The organic phase was dryed with calcium chloride. After filtration the organic solution was evaporated in vacuo to give 0.78 g of the title compound. M.p. 261—69°C.

* Synthesis, Vol. *10,* pp. 681—682.

Example 7

3-(5-cyclopropyl-1,2,4-oxadiazol-3-yl)-5,6-dihydro-5-methyl-6-oxo-7-chloro-4H-imidazo(1,5-a)(1,4)-benzodiazepine.

3,4-dihydro-4-methyl-6-chloro-2H-1,4-benzodiazepine-2,5(1H) dione (USP 4,316,839) (9.17 mmol) was dissolved in dry DMF (20 ml) and charged with sodium hydride (10 mmol). The resulting solution was cooled under $N_2$ to −20°C, whereafter chlordiethylphosphate (11 mmol) was added.

The reaction mixture was kept under $N_2$ with stirring at −20°C and charged with a −30°C cold solution of 5-cyclopropyl-3-isocyanomethyl-1,2,4-oxadiazole (11 mmol) and K-t-butylate (11 mmol) in dry DMF (15 mmol).

The resulting reaction mixture was allowed to heat to room temperature, whereafter it was evaporated to dryness *in vacuo.* The oily residue containing the crude product was purified on $SiO_2$ with ethyl acetate as eluent. This gave the title compound as white crystals.

M.p. 165—168.5°C.

Example 8

3-(5-cyclopropyl-1,2,4-oxadiazol-3-yl)-5,6-dihydro-5-methyl-6-oxo-7-bromo-4H-imidazo(1,5-a)(1,4)-benzodiazepine

2.5 g of 3,4-dihydro-4-methyl-6-bromo-2H-1,4-benzodiazepine-2.5(1H)-dione (USP 4,352,817 ex. 9) (8.5 mmol) was dissolved in dry DMF (30 ml) and charged with 480 mg sodium hydride. The resulting solution was cooled under an atmosphere of $N_2$ to −20°C, whereafter chlordiethyl-phosphate (1.6 ml) was added.

The reaction mixture was kept under $N_2$ with stirring at −20°C and was charged with a −30°C cold solution of 5-cyclopropyl-3-isocyanomethyl-1,2,4-oxadiazol (1.64 g) and K-t-butylate (1.23 g) in dry DMF (15 ml).

The resulting reacting mixture was allowed to heat to room temperature and was stirred for 30 minutes. The crude product was precipitated by addition of 150 ml water. This crude product was partitioned between 50 ml ethylacetate/diethylether (1/1) and 100 ml 4M hydrochloric acid. The organic phase was discharged and the product was precipitated by neutralizing the aqueous phase with 2M sodium hydroxyde. The precipitated product was crystatized from ethylacetate. This gave the title compound as white crystals.

M.p. 212—13°C.

Example 9

3-(5-cyclopropyl-1,2,4-oxadiazol-3-yl)-5,6-dihydro-5-methyl-6-oxo-7-methyl-4H-imidazo(1,5a)(1,4)benzodiazepine

3,4-dihydro-4-methyl-6-methyl-2H-1,4-benzodiazepine-2,5(1H) dione (USP 4,316,839 ex 28) (4.0 mmol) was dissolved in dry DMF (20 ml) and charged with sodium hydride (5 mmol). The resulting solution was cooled under $N_2$ to −20°C, whereafter chlordiethylphosphate (11 mmol) was added.

The reaction mixture was kept under $N_2$ with stirring at −20°C and charged with a −30°C cold solution of 5-cyclopropyl-3-isocyanomethyl-1,2,4-oxadiazole (5 mmol) and K-t-butylate (5 mmol) in dry DMF (15 mmol).

The resulting reaction mixture was allowed to heat to room temperature, whereafter it was evaporated to dryness *in vacuo.* The oily residue containing the crude product was crystallized from ethanol giving 0,45 g of the title compound.

M.p. 175—175.5°C.

The following compounds were synthesized in exactly the same manner.

7-flour-3-(5-cyclopropyl-1,2,4-oxadiazol-3-yl)-5,6-dihydro-5-methyl-6-oxo-4H-imidazo(1,5-a)(1,4)benzodiazepine.

M.p. 188,2—189,0°C.

HNMR (60 mHz, DMSO-$D_6$): δ = 8.2 (s, 1H), 7.2—8.0 (m, 3H aromatic), 5.1—4.2 (degenerate coupling, 2H), 3.1 (s, 3H, NME), 2.3 (m, 1H) 1.1—1.5 (m, 4H), from 3,4-dihydro-4-methyl-6-fluoro-2H-1,4-benzodiazepine-2,5(1H)-dione. M.P. 247,2—247,7°C. Synthesized according to a modified procedure of USP 4,316,839 example 10. The reaction conditions of the reaction step leading to 3,4-dihydro-4-methyl-6-fluoro-2H-1,4-benzodiazepin-2,5(1H)-dione from 6-fluoro-isatoic acid anhydride was 130°C for 8 hours rather than 100°C for 30 minutes, which in repeatedly gave N-methyl-N-(2-amino-6-fluoro-benzoyl)-glycin. Furthermore the melting point reported in USP 4,316,839 for 3,4-dihydro-4-methyl-6-fluoro-2H-benzodiazepin-2-5(1H)-dione has been determined to be significantly different, namely 247,2—247,7°C rather than 214—217°C.

7-cyano-3-(5-cyclopropyl-1,2,4-oxadiazol-3-yl)-dihydro-5-methyl-6-oxo-4H-imidazo (1,5-a)(1,4) benzodiazepine.

M.p. 237—9°C.

IR: 0.3% KBr dish showed the characteristic C≡N band at 2230 $cm^{-1}$ NMR (60 mHz, $CDCl_3$+DMSO-$D_6$): δ = 8.2 (s, 1H), 7.2—8.0 (m, 3H, aromatic), 5.2—4.3 (2m, degenerate coupling, 2H), 3.1 (s, 3H, N—ME), 2.3 (m, 1H), 1.1—1.5 (m, 4H), from 3,4-dihydro-4-methyl-6-cyano-2H-1,4-benzodiazepine-2,5(1H)-dione (Example 6 hereof), 7-trifluoromethyl-3-(5-cyclopropyl-1,2,4-oxodiazol-3-yl)-5,6-dihydro-5-methyl-6-oxo-4H-imidazo (1,5-a)(1,4)benzodiazepine. $H^1$ NMR (60 mHz, DMSO-$D_6$+$CDCL_3$) = (s, 1H), 8.0—7.5 (m, 3H

aromatic), 5.3—4.4 (m, degenerate coupling, 2H), 3.1 (s, 3H, N—ME), 2.3 (m, 1H), 1.1—1.5 (4H), M.p. 200,1°C from 3,4-dihydro-4-methyl-6-trifluoromethyl-2H-1,4-benzodiazepine-2,5(1H) dione. M.p. 224,0°C (prepared exactly as by the methods of US—A—4,316,839).

Example 10

Representative Pharmaceutical Compositions

a) A typical tablet for use in treating anxiety states and which may be prepared by conventional tableting techniques contains

| | |
|---|---|
| Compound of Invention* | 1.0 mg |
| Lactosum (lactose) | 67.4 mg Ph.Eur. |
| Avicel® (microcellulose) | 31.4 mg |
| Amberlite® (IRP 88)** | 1.0 mg |
| Magnesii stearas (magnesium stearate) | 0.25 mg Ph.Eur. |

Exactly the same tablet may be used for treating convulsions.

* possibly salt form
** ion exchange resin

b) For suppositories, any usual suppository base may be employed for incorporation thereinto by usual procedure of the active ingredient, such as a polyethyleneglycol which is a solid at normal room temperature but which melts at or about body temperature. c) For parenteral (including substaneous) sterile solutions, the active ingredient together with conventional ingredients in usual amounts are employed, such as sodium chloride, sodium dihydrogen phosphate, disodium edetate(ethylenediaminetetraacetic acid disodium salt), benzyl alcohol, sodium hydroxide to adjust pH, and double-distilled water q.s., according to conventional procedure, such as filtration, aspetic filling into ampoules, and autoclaving for sterility.

Other suitable pharmaceutical compositions will be immediately apparent to one skilled in the art.

Pharmacology

The compounds of the invention have been found to exhibit an unpredictably favourable and highly advantagenous degree of activity in the standard classical test for determining the *in vivo* affinity for the benzodiazepine receptors, as well as in the standard test considered predictive for pharmaceutical activity against convulsions and anxiety states mediated through the benzodiazepine receptors.

The following test has been performed on some compounds of the invention as well as on representative examples of prior art compounds.

*I. In vivo inhibition of $^3$H-flunitrazepam binding to mouse forebrain membranes by test substances administered intraperitoneally.* (Procedure 130)

*Principle.* Twenty minutes after a dose of $^3$H-flunitrazepam ($^3$H—FNM) (200 μCi/kg i.v.) the amount of specific $^3$H—FNM binding to brain benzodiazepine receptors has reached its maximal value. This specific binding of $^3$H—FNM can be partly or completely prevented by simultaneous or prior administration of pharmacologically active benzodiazepines and by some benzodiazepine-like agents (Chang and Snyder, Eur. J. Pharmacol. *48,* 212—218 (1978)).

*Test procedure.* Suspensions of test substances (2 mg/ml) are prepared in 5% Duphasol-X* ®(TM Duphar; castor oil-ethylene oxide derivative for emulsifying and solubilizing oil and other water-insoluble substances) by sonification for 10 min. using a ®Branson B15 microtip ultrasonifier (setting 7). Groups of three mice (female, NMR, 18—22 gram) are injected with the test substance at 100 mg/kg intraperitorially. Fifteen minutes after test substance administration the mice are challenged with 4 μCi intravenously of $^3$H—FNM (70—90 Ci/mole) in 200 μl physiological saline. Twenty minutes after $^3$H—FNM administration mice are sacrificed by decapitation, the forebrain rapidly excised (within 30 sec) and homogenized in 12 ml of icecold 25 mM $KH_2PO_4$, pH 7.1, using an ®Ultra-Turrax homogenizer fitted with an N 10 shaft. Two aliquots of 1 ml are immediately filtered through Whatman GF/C glassfibre filters and washed with 2 × 5 ml of the above mentioned buffer. The amounts of radioactivity on the filters are determined by conventional scintillation counting. One group of untreated mice serves as controls. One to three mice are injected with 25 mg/kg clonazepam i.p. 30 minutes before $^3$H—FNM to determine the amount of non-specific $^3$H—FNM binding, which should be between 8—15% of total binding.

When doses of 100 mg/kg inhibit more than 50% of specific $^3$H-flunitrazepam binding; test substances are admistered in doses, which are factors of 3.16 times lower than 100 mg/kg.

The $ED_{50}$ for a test substance is defined as the dose which inhibits 50% of specific $^3H$—FNM binding. Specific binding is the amount of binding in controls minus the amount binding in clonazepam-treated mice.

*Results.* The $ED_{50}$ value is determined from dose response curves. If only one dose of test substance is administered the $ED_{50}$ value is calculated as follows, provided that the inhibition of specific binding is within the range of 25—75%:

$$ED_{50} = \text{administered dose} \times \frac{1 \times 1000}{\left(\frac{C_{o-1}}{C_x}\right)} \ \mu g/kg$$

where $C_o$ is specific binding in controls and $C_x$ is specific binding in mice treated with test substance.

*II. Pentazol colonic conv. mice (i.p.)* (Procedure 400)

*Principle.* Pentylenetetrazol induces clonic and tonic convulsions in mice at doses of 60—120 mg/kg s.c. The mechanism is unknown but seems to be due to some effects through the GABA receptor/benzodiazepine receptor/chloride ionophore complex. Antagonism of convulsions induced by maximal doses of pentylenetetrazol is considered predictive for drugs effective against petit mal epilepsia and anxiety.

*Method.* 150 mg/kg pentylenetetrazol dissolved in 0.9% NaCl is given by the subcutaneous route in volumes of 15 ml/kg to male or female NMRI mice weighing 20—25 g 30 min after an intraperitoneal injection of a test compound. Number of mice exhibiting clonic seizures is noted within the next 30 min. At least 3 doses of each test compound are used with 4 to 8 mice per dose, and with doses both above and below the $ED_{50}$ value.

*Results.* The $ED_{50}$ value is calculated as the dose in µg/kg at which seizures are inhibited in 50% of the animals using a computer program based on the method of Litchfield and Wilcoxon (1949).

*III. Pentazol tonic conv. mice i.p.* (Procedure 401)

*Principle.* Pentylenetetrazol induces clonic and tonic convulsions in mice at doses of 60—120 mg/kg s.c. The mechanism is unknown but seem to be due to some effects through the GABA receptor/benzodiazepine receptor/chloride ionophore complex. Antagonism of convulsions induced by maximal doses of pentylenetetrazol is considered predictive for drugs effective against petit mal epilepsia and anxiety.

*Method.* 150 mg/kg pentylenetetrazol (Pentazol, Sigma) dissolved in 0.9% NaCl is given by the subcutaneous route in volumes of 15 mg/kg to male or female NMRI mice weighing 20—25 g 30 min after an intraperitoneal injection of a test compound. Number of mice exhibiting tonic seizures is noted within the next 30 min. At least 3 doses of each test compound are used with 4 to 8 mice per dose, and with doses both above and below the $ED_{50}$ value.

*Results.* The $ED_{50}$ value is calculated as the dose in µg/kg where seizures are inhibited in 50% of the animals using a computer program based on the method of Litchfield and Wilcoxon (1949).

THE TABLE

Test results obtained by testing the compound of the invention and the compounds considered to be the closest prior art will appear from the following table 1.

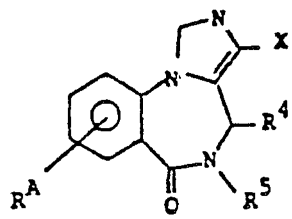

| | 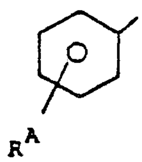 | $R^4$ | $R^5$ | X | in vivo binding ED50µg/kg | Activity against pentazol induced convulsions ED50 µg/kg clonic | tonic |
|---|---|---|---|---|---|---|---|
| A | 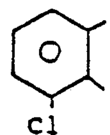 | H | $CH_3$ | 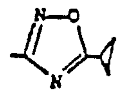 | 27 | 40 | 3 |
| A | 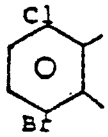 | H | $CH_3$ | 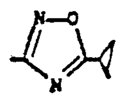 | 15 | 2 | 0,2 |
| A | 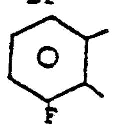 | H | $CH_3$ | 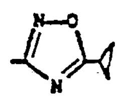 | 90 | 20 | 5 |
| A | 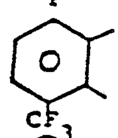 | H | $CH_3$ | 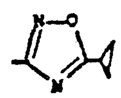 | 10 | 8 | 3 |
| B | 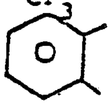 | H | $CH_3$ | 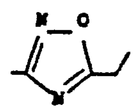 | 1.800 | 27.000 | 1.300 |
| C | 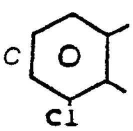 | H | $CH_3$ | 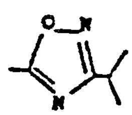 | 200 | 600 | 200 |
| D | 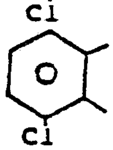 | H | $CH_3$ | 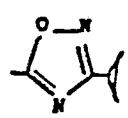 | 60 | 90 | 30 |
| E | 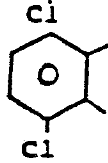 | $-CH_2CH_2CH_2-$ | | 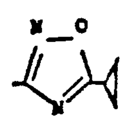 | 960 | 13.000 | 1.000 |
| F | 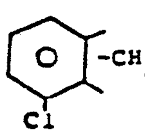 | $-CH_2CH_2CH_2-$ | | 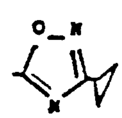 | 230 | 1.600 | 80 |
| G | 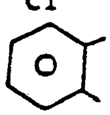 | H | $CH_3$ | 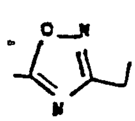 | 2.200 | 8.000 | 2.000 |

A Compound of the present invention
B Compound of EP—A—109921 ex. 3
C Compound of EP—A—109921 page 3, line 5
D Compound of EP—A—150040 ex. 29
E Compound of EP—A—150040 ex. 40
F Compound of EP—A—150040 ex. 31
G Compound of EP—A—109921 ex. 1

From above table it is readily apparent that the compound of the invention is remarkably and unpredictably superior in every respect compared to the most structurally closely-related compounds of the prior art.

In direct comparison to its corresponding 3-ethyl-1,2,4-oxadiazol-5-yl analogue (G) of EP—A—109921 (ex. 1) the compound (B) of EP—A—109921 (ex. 3) has about the same activity and it can be concluded that the 5-ethyl-1,2,4-oxadiazol-3-yl) substituent is about equivalent to the corresponding 3-ethyl-1,2,4-oxadiazol-5-yl substituent.

In direct comparison to its 3-cyclopropyl-1,2,4-oxadiazol-5-yl analogue (F) of EP—A—150040 (ex. 31) the corresponding 5-cyclopropyl-1,2,4-oxadiazol-3-yl (E) compound of EP—A—150040 (ex. 40) is significantly inferior and it can be concluded that 5-cyclopropyl-1,2,4-oxadiazol-3-yl is a highly inferior embodiment of EP—A—150040.

In direct comparison to its corresponding 3-cyclopropyl-1,2,4-oxadiazol-5-yl analogue (D) of EP—A—150040 (ex. 29) the chloro compound of the invention is more than 2 times as active in binding affinity for the benzodiazepine receptor, approximately 2¼ times a active in protecting against pentetrazol induced clonic convulsions, and 10 times as active in protecting against pentetrazol induced tonic convulsions.

In direct comparison to the compound E having exactly the same substituted oxadiazol-3-yl substituent the chloro compound of the invention is 35 times more as active binding affinity for the benzodiazepine receptor, 325 times as active in convulsions, and 330 times as active in protecting against pentetrazol induced tonic convulsions.

In direct comparison to the compound E having exactly the same substituted oxadiazol-3-yl substituent the bromo compound of the invention is 60 times more as active in binding affinity, 6500 times as active in protecting against pentetrazol induced clonic convulsions, and 500 times as active in protecting against pentetrazol induced tonic convulsions.

In conclusion, from the foregoing, it is apparent that the present invention provides a novel anticonvulsant and anxiolytic 3-(5-cyclopropyl-1,2,4-oxadiazol-3-yl)-5,6-dihydro-5-methyl-6-oxo-7-x-4H-imidazo (1,5-a)(1,4) benzodiazepines and addition salts thereof, having highly advantageous and unpredictable properties.

Further, a new synthesis is provided by the present invention.

**Claims**

1. 3-(5-Cyclopropyl-1,2,4-oxadiazol-3-yl)-5,6-dihydro-5-methyl-6-oxo-7-X-4H-imidazo (1,5-a) (1,4) benzodiazepines having the formula

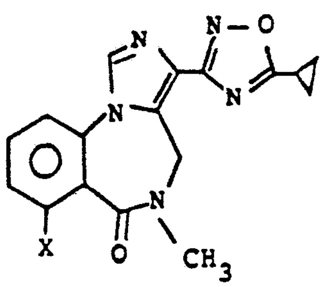

wherein X is Cl, Br, F, $CF_3$, $CH_3$ or CN, and pharmaceutically acceptable acid addition salts thereof.

2. A pharmaceutical composition suitable for use in the treatment of a central nervous system ailment comprising an amount of a compound of claim 1 which is effective for the alleviation of such disorder together with a pharmaceutically acceptable carrier or diluent.

3. The method of preparing the compounds according to claim 1 which comprises,

a) the step of reacting a compound having the formula

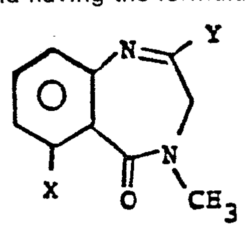

wherein X is Cl, Br, F, $CF_3$, $CH_3$, CN and wherein Y is a leaving group, with a compound of the formula

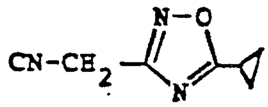

to produce the desired compound or

b) reacting a compound having the formula

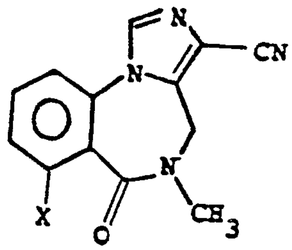

wherein X has the meanings set forth above with $NH_2OH$ to form a compound having the formula

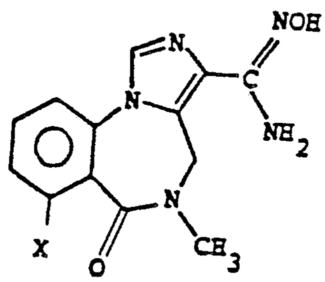

wherein X has the meanings set forth above and reacting the compound thus obtained with 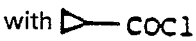 to form a compound of the invention.

4. Method of Claim 3, wherein in step a) the reaction is carried out under alkaline conditions.

5. Method of Claim 3, wherein in step a) the reaction is carried out in the presence of an organic solvent.

6. Method of claim 3, 4 or 5 wherein the 7-X-substituent in the starting compound and end products is 7-chloro, 7-bromo, 7-fluoro, 7-$CF_3$ or 7-CN.

**Patentansprüche**

1. 3-(5-Cyclopropyl-1,2,4-oxadiazol-3-yl)-5,6-dihydro-5-methyl-6-oxo-7-X-4H-imidazo (1,5a) (1,4) benzodiazepine der allgemeinen Formel

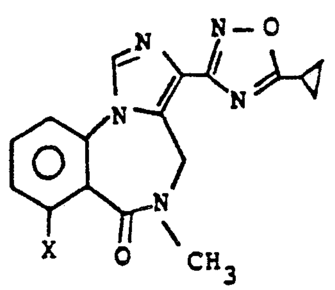

worin X Cl, Br, F, $CF_3$, $CH_3$ oder CN bedeutet, und pharmazeutisch annehmbare Säureadditionssalze hiervon.

2. Pharmazeutische Zusammensetzung, die zur Behandlung eines Leidens des Zentralnervensystems geeignet ist, umfassend eine Verbindung nach Anspruch 1 in einer zur Linderung eines solchen Leidens wirksamen Menge, zusammen mit einem pharmazeutisch annehmbare Träger oder Verdünnungsmittel.

3. Verfahren zur Herstellung der Verbindungen nach Anspruch 1, umfassend

a) die Stufe des Umsetzens einer Verbindung der Formel

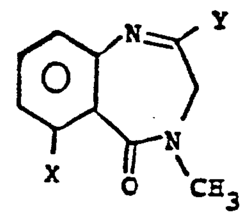

worin X Cl, Br, F, $CF_3$, $CH_3$, CN und Y eine Abspaltgruppe bedeuten, mit einer Verbindung der Formel

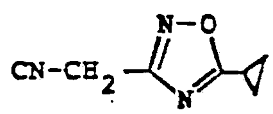

um die erwünschte Verbindung herzustellen oder

b) das Umsetzen einer Verbindung der Formel

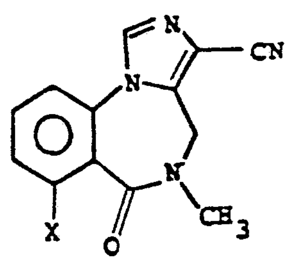

worin X die oben angegebenen Bedeutungen besitzt mit $NH_2OH$, zur Bildung einer Verbindung der Formel

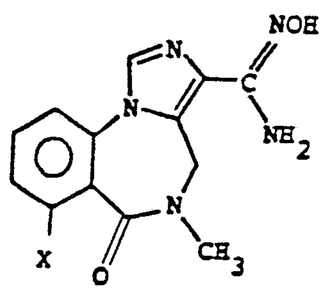

worin X die oben angegebenen Bedeutungen besitzt, und Umsetzen der so erhaltenen Verbindung mit

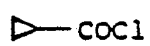

um eine erfindungsgemäße Verbindung zu bilden.

4. Verfahren nach Anspruch 3, wobei in Stufe a) die Umsetzung unter alkalischen Bedingungen durchgeführt wird.

5. Verfahren nach Anspruch 3, wobei in Stufe a) die Umsetzung in Gegenwart eines organischen Lösungsmittels durchgeführt wird.

6. Verfahren nach Anspruch 3, 4 oder 5, wobei der 7-X-Substituent in der Ausgangsverbindung und den Endprodukten 7-Chlor, 7-Brom, 7-Fluor, 7-$CF_3$oder 7-CN ist.

**Revendications**

1. 3-(5-cyclopropyl-1,2,4-oxadiazol-3-yl)-5,6-dihydro-5-méthyl-6-oxo-7-X-4H-imidazo(1,5-a)(1,4)benzodiazépines répondant à la formule:

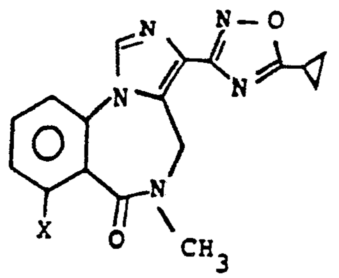

dans laquelle X est Cl, Br, F, $CF_3$, $CH_3$ ou CN, et leurs sels d'addition acides pharmacetiquement acceptables.

2. Composition pharmaceutique utilisable pour le traitement d'une affection du système nerveux central, comprenant une quantité d'un composé suivant la revendication 1 qui est efficace pour le soulagement de cette affection, en même temps qu'un support ou diluant pharmaceutiquement acceptable.

3. Procédé de préparation des composés suivant la revendication 1, qui comprend:

a) le stade consistant à faire réagir un composé répondant à la formule:

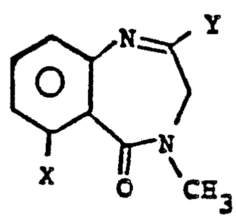

dans laquelle X est Cl, Br, F, $CF_3$, CN et dans laquelle Y est un groupe partant, avec un composé répondant à la formule:

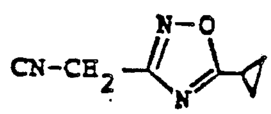

pour préparer le composé désiré, ou

b) le stade consistant à faire réagir un composé répondant à la formule:

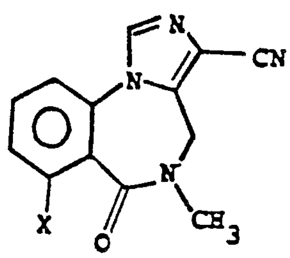

dans laquelle X a les significations indiquées ci-dessus avec $NH_2OH$ pour former un composé répondant à la formule:

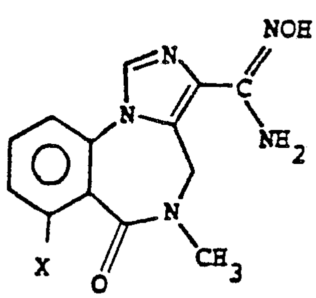

dans laquelle X a les significations indiquées ci-dessus, et à faire réagir le composé ainsi obtenu avec

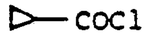

pour former un composé de l'invention.

4. Procédé de la revendication 3, dans lequel dans l'étape a) la réaction est effectuée en milieu alcalin.

5. Procédé de la revendication 3, dans lequel dans l'étape a) la réaction est effectuée en présence d'un solvant organique.

6. Procédé des revendications 3, 4 ou 5, dans lequel le substituant 7-X dans le composé de départ et les produits finals est un groupe 7-chloro, 7-bromo, 7-fluoro, 7-$CH_3$ ou 7-CN.